# EUROPEAN PATENT SPECIFICATION

(11) **EP 4 405 020 B1**
(45) Date of publication and mention of the grant of the patent: **29.10.2025**
(21) Application number: 22772681.7
(22) Date of filing: 23.08.2022
(51) Int. Cl.: A61M 16/20, A61M 13/00, A61M 16/00, A61B 17/34

(54) **INSUFFLATOR SAFETY VALVE HAVING ELECTRICALLY VARIABLE CRACKING PRESSURE**
SICHERHEITSVENTIL FÜR INSUFFLATOR MIT ELEKTRISCH VARIABLEM SPALTDRUCK
SOUPAPE DE SÉCURITÉ D'INSUFFLATEUR AYANT UNE PRESSION DE CRAQUAGE ÉLECTRIQUEMENT VARIABLE

(30) Priority: 20.09.2021 US 202163246018 P
(43) Date of publication of application: 31.07.2024
(73) Proprietor: CONMED Corporation, Largo, FL 33773 (US)
(72) Inventor: KOLTZ, Michael L., Jr., Aurora, CO 80016 (US)
(74) Representative: Hoefer & Partner Patentanwälte mbB
(86) International application number: PCT/US2022/041170
(87) International publication number: WO 2023/043586

(56) References cited:
- WO-A1-2020/249813
- US-A1- 2014 012 184
- US-A1- 2014 309 583
- US-A1- 2021 267 639

## Description

### CROSS-REFERENCE TO RELATED APPLICATIONS

The subject application claims the benefit of priority to U.S. Provisional Patent Application Serial No. 63/246,018 filed September 20, 2021.

### BACKGROUND OF THE DISCLOSURE

### 1. Field of the Disclosure

The subject application is directed to endoscopic surgery, and more particularly, to an insufflator for delivering surgical gas to a patient's body cavity, which includes a safety valve having electrically variable cracking pressure.

### 2. Description of Related Art

Laparoscopic or "minimally invasive" surgical techniques are becoming commonplace in the performance of procedures such as cholecystectomies, appendectomies, hernia repair and nephrectomies. These procedures commonly involve filling or "insufflating" the abdominal cavity with a pressurized fluid, such as carbon dioxide, to create an operating space, which is referred to as a pneumoperitoneum.

Insufflation associated with laparoscopic surgery presents an inherent patient risk associated with over-pressurization of the abdominal cavity. Over-pressurization has led to numerous patient injuries in both adult and pediatric victims. Most insufflators in use today incorporate a solely mechanical safety valve plumbed into the intermediate pressure node of the gas insufflation network. The safety valve serves as a final means of protection against mechanical, electrical, and software failures which could result in pressure exceeding a pneumoperitoneal control set-point.

While insufflator safety valves known in the prior art are adjustable, they are typically fixed to a specified value or cracking pressure only at the time of manufacture or subsequent service. This non-user-adjustable nature of the valve requires it to be set above maximum normal expected pressure levels to prevent the valve from intermittently leaking during surgical use. These leak preventative pressure set-points are commonly above the threshold for mitigating over-pressurization in most applications when used on a majority of the patient population.

It would be beneficial to provide an insufflator safety valve wherein the cracking pressure or set-point of the valve that can be adjusted intra-operatively. This adjustability would enable cracking pressures to be set much lower than safety valves described in the prior art, without frequent valve opening. Furthermore, the cracking pressure could be lowered for surgeries requiring less gas flow or for patients with smaller peritoneal volume, such as children.

The following documents disclose insufflators having electrically variable safety valves, namely US 2021/267639 A1, US 2014/309583 Al and WO 2020/249813 A1.

### SUMMARY OF THE DISCLOSURE

The invention is defined in the appended claims.

The subject disclosure is directed to a new and useful insufflator for delivering surgical gas from a gas source to a patient's body cavity during a surgical procedure. The insufflator includes an electrically variable safety valve that is adapted and configured to prevent over-pressurization of the patient's body cavity during the surgical procedure by venting the surgical gas in the patient's body cavity to atmosphere when a cracking pressure of the variable safety valve is exceeded. In use, when a pressure differential across the valve exceeds the cracking pressure, the valve is "cracked open" and gas can flow.

In accordance with the subject disclosure, the variable safety valve includes an electric motor for intra-operatively adjusting the cracking pressure of the variable safety valve, a motor controller for commanding the electric motor to adjust the cracking pressure of the variable safety valve based upon a feedback signal. In one embodiment of the subject disclosure, the feedback signal is provided by an input signal from an operator. In another embodiment, the feedback signal is provided by a signal from a sensor.

When the input signal is provided by an operator, the motor controller will command the electric motor to adjust the cracking pressure of the variable safety valve based on an operator selected cracking pressure selected from a set of at least two discrete cracking pressure set points. When the signal is provided by a sensor, the motor controller commands the electric motor to adjust cracking pressure of the variable safety valve based upon a feedback signal from a flow sensor and/or a pressure sensor.

The insufflator further includes a flow sensor for monitoring a flow rate of the surgical gas delivered to the patient's body cavity, a pressure sensor for monitoring the pressure of the surgical gas delivered to the patient's body cavity, a control valve for controlling the delivery of surgical gas from the insufflator to the patient's body cavity, and a pressure regulator for controlling the pressure of surgical gas delivered to the insufflator from the gas source.

In accordance with the subject disclosure, the electric motor is a stepper motor that drives a lead nut which translates rotary motion into linear motion by way of a lead screw. The lead screw drives a pressure plate in contact with one end of a valve spring, effectively changing an installed length of the valve spring. The shortening of the installed length of the valve spring linearly correlates with increasing the cracking pressure of the variable safety valve, which permits gas flow when the gas pressure acting against a front face of the diaphragm exceeds a spring force applied to a back side of the diaphragm.

These and other features of the electrically variable insufflator safety valve of the subject disclosure will become more readily apparent from the following detailed description of the preferred embodiments taken in conjunction with the drawings.

### BRIEF DESCRIPTION OF THE DRAWINGS

So that those skilled in the art will readily understand how to make and use the insufflator safety valve of the subject disclosure without undue experimentation, embodiments thereof will be described in detail herein below with reference to the figures wherein:
Fig. 1 is a schematic representation of an insufflator constructed in accordance with subject disclosure; and
Fig. 2 is a cross-sectional view of the electrically variable safety valve of the subject disclosure, which is incorporated into the insufflator shown in Fig. 1.

### DESCRIPTION OF THE DISCLOSED EMBODIMENTS

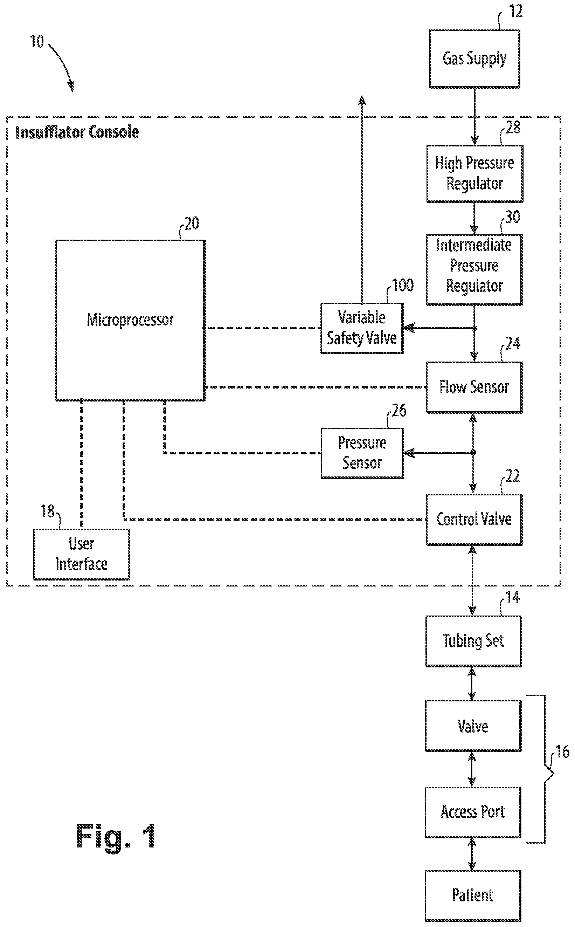

Referring now to the drawings wherein like reference numeral identify similar features or components of the subject disclosure, there is illustrated in Fig. 1 a schematic representation of an insufflator console 10 incorporating the variable safety valve 100 of the subject disclosure. The insufflator console 10 receives pressurized surgical gas from a gas supply source 12 and delivers pressure regulated surgical gas to a patient's body cavity by way of a tube set 14 that communicates with a valve sealed access port or trocar 16.

The insufflator 10 includes a graphical user interface 18 or GUI and a microprocessor or motor controller 20 for controlling the variable safety valve 100 and a separate flow control valve 22 based on user inputs through the GUI 18 and feedback signals from a flow sensor 24 and/or a pressure sensor 26.

In use, surgical gas entering the insufflator console 10 from gas supply 12 passes initially through a high pressure regulator 28 and then through an intermediate pressure regulator 30. The gas flow then interacts with the flow sensor 24 and pressure sensor 26 before flowing to the control valve 22, which moderates or otherwise controls the delivery of gas from the insufflator console 10 to the tube set 14 and attached to the valve sealed access port 16.

The variable safety valve 100 of the subject disclosure is positioned downstream from the intermediate pressure regulator 30 and upstream from the flow sensor 24. It is adapted and configured to direct or otherwise vent gas from the patient's abdominal cavity, out of the insufflator console 10 and into the surrounding atmosphere when it is actuated, so as to advantageously prevent over-pressurization of the patient's abdominal cavity. As explained in more detail below, the variable safety valve 100 is actuated when a pressure differential across the valve exceeds the valves' cracking pressure or pneumoperitoneal control set-point.

It is envisioned that the variable safety valve 100 of the subject disclosure could be incorporated into the low pressure insufflation manifold of the multi-modal gas delivery system disclosed in commonly assigned U.S. Patent Application Publication 2022/0233791.

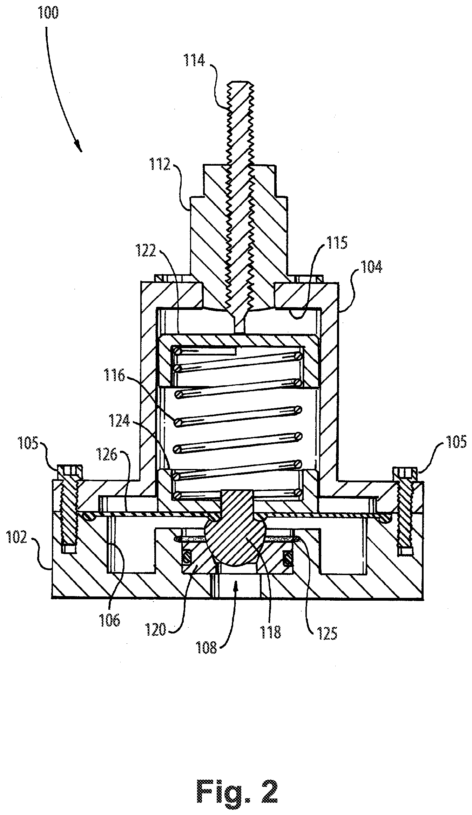

Referring now to Fig. 2, the variable safety valve includes 100 a base 102 and a housing 104. The housing is connected to the base 102 by threaded fasteners 105. The base 102 of valve 100 has an interior pressure cavity 106 that communicates with the gas delivery flow path of insufflator 10, downstream from the intermediate pressure regulator 30 and upstream from the flow sensor 24, as illustrated in Fig. 1. The base 102 also has a vent port 108, which communicates with the pressure cavity 106 and with the surrounding atmosphere.

The flow control element of safety valve 100 is a poppet valve member 118, which is associated with a valve seat 120 located between the pressure cavity 106 and the vent port 108 and secured in place by a lock ring 125. The poppet valve member 118 is operatively associated with a flexible diaphragm 126 that extends across the interior pressure cavity 106 and is securely fixed about its outer periphery between the base 102 and valve housing 104. The front face of the diaphragm 126 defines an upper boundary of the pressure cavity 106 and it will react to pressure changes therein.

The rear or back face of the diaphragm 126 is operatively associated with a coiled valve spring 116 supported with the interior chamber 115 of housing 104 of valve 100. The coiled valve spring 116 is sandwiched between an upper pressure plate 122 and a lower pressure plate 124. The lower pressure plate 124 is operatively associated with the back face of diaphragm 126 while the upper pressure plate 122 is operatively connected to an electric motor (e.g., a stepper motor) 112. The upper pressure plate 122 is connected to the electric motor 112 by way of a torque/force multiplying drive mechanism in the form of a lead screw 114.

Those skilled in the art will readily appreciate that the motor 112 need not be a stepper motor, in that any electric motor with position feedback can be substituted. The stepper motor is desirable however, as position can be controlled by step inputs eliminating the need for a position feedback loop. Those skilled in the art will also appreciate that the drive mechanism need not be direct or translate to linear motion. Alternatives incorporating gears, rotary actuation, and non-linear springs are also envisioned and well within the scope of the subject disclosure.

In use, when the cracking pressure of the valve 100 is adjusted intra-operatively, the stepper motor 112 drives a lead nut (not shown) which translates rotary motion into linear motion by way of the lead screw 114. The lead screw motion drives the upper pressure plate 122 in contact with the upper end of valve spring 116, which effectively changes the installed length of the valve spring 116 within valve housing 104. Shortening the installed length of the valve spring 116 linearly correlates with increasing the cracking pressure of the safety valve 100, which permits gas flow when the gas pressure acting within pressure chamber 106 against the front face of the diaphragm 126 exceeds the spring force applied to the back side of the diaphragm 126 by the valve spring 116.

In one embodiment of the subject disclosure, a user or operator can select a cracking pressure for the valve 100 from at least two discrete cracking pressure set points by way of the graphical user interface 18 of the insufflator 10. In another embodiment of the subject disclosure, the operator can continuously chose from a group of selectable set points throughout a cracking pressure range. In yet another embodiment of the subject disclosure, the cracking pressure of the valve 100 is continuously and automatically set by the processor/motor controller 20 based on a feedback signal from the flow sensor 24 and/or the pressure sensor 26. This automated control facilitated by processor 20 can be implemented by way of appropriate electrical circuitry with or without associated software.

The subject disclosure eliminates the user-based set-point decision process and enables the insufflation system to actively adapt to downstream configurations (e.g., tubes, valves, access ports, etc.) and patient variation by monitoring pressure, flow, or a combination thereof. For example, the insufflation system can set the initial cracking pressure to a low cracking pressure of 5 mmHg to maintain a high level of over-pressurization safety and automatically increase the cracking pressure set-point as the measured gas delivery rate at flow sensor 24 increases or the measured intermediate node pressure increases at pressure sensor 26, while never permitting the set-point to exceed the maximum 80 mmHg level or a lower user selected/configurable value.

Under this scenario, if a fault occurs leading to an over-pressurization hazard, the set point or cracking pressure would never be above the 80 mmHg threshold, and when considered as a fluctuating value over time, the cracking pressure at the time of the fault would likely be closer to the pneumoperitoneal set point rather than the 80 mmHg upper limit.

While the subject disclosure has been shown and described with reference to preferred embodiments, those skilled in the art will readily appreciate that changes and/or modifications may be made thereto without departing from the scope of the subject disclosure.

## Claims

1. An insufflator (10) for delivering surgical gas from a gas source to a patient's body cavity during a surgical procedure, comprising:
an electrically variable safety valve (100) adapted and configured to prevent over-pressurization of the patient's body cavity during the surgical procedure by venting the surgical gas in the patient's body cavity to atmosphere when a cracking pressure of the variable safety valve (100) is exceeded, and
wherein the variable safety valve includes an electric motor (112) for intra-operatively adjusting the cracking pressure of the variable safety valve (100), and
**characterized in that** the electric motor (112) is a stepper motor that translates rotary motion into linear motion by way of a lead screw (114),

2. An insufflator (10) as recited in Claim 1, further comprising a motor controller (20) for commanding the electric motor (112) to adjust the cracking pressure of the variable safety valve (100) based upon a feedback signal.

3. An insufflator (10) as recited in Claim 2, wherein the feedback signal is provided by an input signal from an operator.

4. An insufflator (10) as recited in Claim 2, wherein the feedback signal is provided by a signal from a sensor.

5. An insufflator (10) as recited in Claim 3, wherein the motor controller (20) is configured to command the electric motor (112) to adjust the cracking pressure of the variable safety valve (100) based on an operator selected cracking pressure selected from a set of at least two discrete cracking pressure set points.

6. An insufflator (10) as recited in Claim 4, wherein the motor controller (20) commands the electric motor (112) to adjust cracking pressure of the variable safety valve (100) based upon a feedback signal from a flow sensor and/or a pressure sensor.

7. An insufflator (10) as recited in Claim 6, further comprising a flow sensor (24) for monitoring a flow rate of the surgical gas delivered to the patient's body cavity.

8. An insufflator (10) as recited in Claim 6, further comprising a pressure sensor (26) for monitoring the pressure of the surgical gas delivered to the patient's body cavity.

9. An insufflator (10) as recited in Claim 1, further comprising a control valve (22) for controlling the delivery of surgical gas from the insufflator (10) to the patient's body cavity.

10. An insufflator (10) as recited in Claim 1, further comprising a pressure regulator for controlling the pressure of surgical gas delivered to the insufflator (10) from the gas source.

11. An insufflator (10) as recited in Claim 1, wherein the lead screw (114) drives a pressure plate (124) in contact with one end of a valve spring (116), effectively changing an installed length of the valve spring (116).

12. An insufflator (10) as recited in Claim 11, wherein shortening the installed length of the valve spring (116) linearly correlates with increasing the cracking pressure of the variable safety valve (100), which permits gas flow when the gas pressure acting against a front face of the diaphragm (126) exceeds a spring force applied to a back side of the diaphragm (126).

13. An insufflator (10) as recited in claim 3,
wherein the motor controller (20) is configured to command the electric motor (112) to adjust the cracking pressure of the variable safety valve (100) based upon an operator selected cracking pressure.

## Patentansprüche

1. Insufflator (10) zum Zuführen von chirurgischem Gas von einer Gasquelle zu einer Körperhöhle eines Patienten während eines chirurgischen Eingriffs, umfassend:
ein elektrisch variables Sicherheitsventil (100), das angepasst und konfiguriert ist, um eine Überdruckbeaufschlagung der Körperhöhle des Patienten während des chirurgischen Eingriffs zu verhindern, indem das chirurgische Gas in der Körperhöhle des Patienten in die Atmosphäre entlüftet wird, wenn ein Rissdruck des variablen Sicherheitsventils (100) überschritten wird, und
wobei das variable Sicherheitsventil einen Elektromotor (112) zum intraoperativen Einstellen des Rissdrucks des variablen Sicherheitsventils (100) beinhaltet, und
**dadurch gekennzeichnet, dass** der Elektromotor (112) ein Schrittmotor ist, der eine Drehbewegung mittels einer Leitspindel (114) in eine lineare Bewegung umsetzt.

2. Insufflator (10) nach Anspruch 1, ferner umfassend eine Motorsteuerung (20) zum Anweisen des Elektromotors (112), den Rissdruck des variablen Sicherheitsventils (100) basierend auf einem Rückkopplungssignal einzustellen.

3. Insufflator (10) nach Anspruch 2, wobei das Rückkopplungssignal durch ein Eingangssignal von einem Bediener bereitgestellt wird.

4. Insufflator (10) nach Anspruch 2, wobei das Rückkopplungssignal durch ein Signal von einem Sensor bereitgestellt wird.

5. Insufflator (10) nach Anspruch 3, wobei die Motorsteuerung (20) konfiguriert ist, um den Elektromotor (112) anzuweisen, den Rissdruck des variablen Sicherheitsventils (100) basierend auf einem vom Bediener ausgewählten Rissdruck einzustellen, der aus einem Satz von mindestens zwei diskreten Rissdrucksollwerten ausgewählt ist.

6. Insufflator (10) nach Anspruch 4, wobei die Motorsteuerung (20) den Elektromotor (112) anweist, den Rissdruck des variablen Sicherheitsventils (100) basierend auf einem Rückkopplungssignal von einem Durchflusssensor und/oder einem Drucksensor einzustellen.

7. Insufflator (10) nach Anspruch 6, ferner umfassend einen Durchflusssensor (24) zum Überwachen einer Durchflussrate des chirurgischen Gases, das der Körperhöhle des Patienten zugeführt wird.

8. Insufflator (10) nach Anspruch 6, ferner umfassend einen Drucksensor (26) zum Überwachen des Drucks des chirurgischen Gases, das der Körperhöhle des Patienten zugeführt wird.

9. Insufflator (10) nach Anspruch 1, ferner umfassend ein Steuerventil (22) zum Steuern der Zufuhr von chirurgischem Gas von dem Insufflator (10) zu der Körperhöhle des Patienten.

10. Insufflator (10) nach Anspruch 1, ferner umfassend einen Druckregler zum Steuern des Drucks des chirurgischen Gases, das dem Insufflator (10) von der Gasquelle zugeführt wird.

11. Insufflator (10) nach Anspruch 1, wobei die Leitspindel (114) eine Druckplatte (124) in Kontakt mit einem Ende einer Ventilfeder (116) antreibt, wodurch eine installierte Länge der Ventilfeder (116) effektiv geändert wird.

12. Insufflator (10) nach Anspruch 11, wobei das Verkürzen der installierten Länge der Ventilfeder (116) linear mit dem Erhöhen des Rissdrucks des variablen Sicherheitsventils (100) korreliert, was einen Gasstrom ermöglicht, wenn der Gasdruck, der gegen eine Vorderseite der Membran (126) wirkt, eine Federkraft überschreitet, die auf eine Rückseite der Membran (126) ausgeübt wird.

13. Insufflator (10) nach Anspruch 3,
wobei die Motorsteuerung (20) konfiguriert ist, um den Elektromotor (112) anzuweisen, den Rissdruck des variablen Sicherheitsventils (100) basierend auf einem vom Bediener ausgewählten Rissdruck einzustellen.

## Revendications

1. Insufflateur (10) pour délivrer un gaz chirurgical d'une source de gaz à une cavité corporelle d'un patient pendant une procédure chirurgicale, comprenant :
une soupape de sécurité électriquement variable (100) adaptée et configurée pour empêcher une surpressurisation de la cavité corporelle du patient pendant la procédure chirurgicale en évacuant le gaz chirurgical dans la cavité corporelle du patient dans l'atmosphère lorsqu'une pression de fissuration de la soupape de sécurité variable (100) est dépassée, et
dans lequel la soupape de sécurité variable comprend un moteur électrique (112) pour ajuster de manière intra-opératoire la pression de fissuration de la soupape de sécurité variable (100), et
**caractérisé en ce que** le moteur électrique (112) est un moteur pas à pas qui convertit un mouvement rotatif en un mouvement linéaire au moyen d'une vis-mère (114).

2. Insufflateur (10) selon la revendication 1, comprenant en outre un dispositif de commande de moteur (20) pour commander le moteur électrique (112) pour ajuster la pression de fissuration de la soupape de sécurité variable (100) sur la base d'un signal de rétroaction.

3. Insufflateur (10) selon la revendication 2, dans lequel le signal de rétroaction est fourni par un signal d'entrée provenant d'un opérateur.

4. Insufflateur (10) selon la revendication 2, dans lequel le signal de rétroaction est fourni par un signal provenant d'un capteur.

5. Insufflateur (10) selon la revendication 3, dans lequel le dispositif de commande de moteur (20) est configuré pour commander le moteur électrique (112) pour ajuster la pression de fissuration de la soupape de sécurité variable (100) sur la base d'une pression de fissuration sélectionnée par l'opérateur sélectionnée parmi un ensemble d'au moins deux points de consigne de pression de fissuration distincts.

6. Insufflateur (10) selon la revendication 4, dans lequel le dispositif de commande de moteur (20) commande le moteur électrique (112) pour ajuster la pression de fissuration de la soupape de sécurité variable (100) sur la base d'un signal de rétroaction provenant d'un capteur de débit et/ou d'un capteur de pression.

7. Insufflateur (10) selon la revendication 6, comprenant en outre un capteur de débit (24) pour surveiller un débit du gaz chirurgical délivré à la cavité corporelle du patient.

8. Insufflateur (10) selon la revendication 6, comprenant en outre un capteur de pression (26) pour surveiller la pression du gaz chirurgical délivré à la cavité corporelle du patient.

9. Insufflateur (10) selon la revendication 1, comprenant en outre une soupape de commande (22) pour commander la délivrance de gaz chirurgical de l'insufflateur (10) à la cavité corporelle du patient.

10. Insufflateur (10) selon la revendication 1, comprenant en outre un régulateur de pression pour commander la pression du gaz chirurgical délivré à l'insufflateur (10) à partir de la source de gaz.

11. Insufflateur (10) selon la revendication 1, dans lequel la vis-mère (114) entraîne une plaque de pression (124) en contact avec une extrémité d'un ressort de soupape (116), changeant efficacement une longueur installée du ressort de soupape (116).

12. Insufflateur (10) selon la revendication 11, dans lequel un raccourcissement de la longueur installée du ressort de soupape (116) est linéairement corrélé à une augmentation de la pression de fissuration de la soupape de sécurité variable (100), qui permet un écoulement de gaz lorsque la pression de gaz agissant contre une face avant du diaphragme (126) dépasse une force de ressort appliquée à un côté arrière du diaphragme (126).

13. Insufflateur (10) selon la revendication 3,
dans lequel le dispositif de commande de moteur (20) est configuré pour commander le moteur électrique (112) pour ajuster la pression de fissuration de la soupape de sécurité variable (100) sur la base d'une pression de fissuration sélectionnée par l'opérateur.
